# EUROPÄISCHE PATENTSCHRIFT

(11) **EP 2 384 362 B1**
(45) Veröffentlichungstag und Bekanntmachung des Hinweises auf die Patenterteilung: **10.10.2012**
(21) Anmeldenummer: 09767964.1
(22) Anmeldetag: 02.12.2009
(51) Int. Cl.: C12M 1/12, C12M 1/22, C12M 1/26

(54) **TRANSFEREINHEIT UND VERFAHREN ZUR AUFNAHME EINES MEDIUMS AUS EINER BEHANDLUNGSVORRICHTUNG**
TRANSFER UNIT AND METHOD FOR RECEIVING A MEDIUM FROM A PROCESSING DEVICE
UNITÉ DE TRANSFERT ET PROCÉDÉ POUR PRENDRE UN SUPPORT D'UN DISPOSITIF DE TRAITEMENT

(30) Priorität: 12.01.2009 DE 102009004667
(43) Veröffentlichungstag der Anmeldung: 09.11.2011
(73) Patentinhaber: Sartorius Stedim Biotech GmbH, 37079 Göttingen (DE)
(72) Erfinder: PFLANZ, Karl, 37130 Reinhausen (DE); GRAUS, Andreas, 37176 Nörten-Hardenberg (DE)
(74) Vertreter: Schneider, Peter Christian
(86) Internationale Anmeldenummer: PCT/EP2009/008574
(87) Internationale Veröffentlichungsnummer: WO 2010/078891

(56) Entgegenhaltungen:
- WO-A1-2008/113443
- WO-A1-2008/113444
- DE-A1- 19 823 993
- DE-A1-102008 005 968
- DE-B3-102005 008 220
- DE-C1- 4 220 560
- DE-U1-202004 001 703
- US-A- 4 668 633
- US-A1- 2002 096 468

## Beschreibung

### Gebiet der Erfindung

Die Erfindung betrifft eine Transfereinheit zur Aufnahme eines insbesondere porösen, scheibenförmigen Mediums aus einer ersten Behandlungsvorrichtung mit mindestens einem Oberteil, wobei das Oberteil einen Fixierrand aufweist, der zur Entnahme des Mediums aus der ersten Behandlungsvorrichtung mit einem Rand des Mediums verbindbar ist.

Weiterhin betrifft die Erfindung ein Verfahren zur mikrobiologischen Untersuchung und/oder Behandlung flüssiger Proben, bei dem ein Oberteil einer Transfereinheit auf ein in einem ersten Unterteil einer ersten Behandlungsvorrichtung angeordnetes und der flüssigen Probe ausgesetztes scheibenförmiges Medium so aufgesetzt wird, dass ein in dem Oberteil angeordneter Fixierrand mit einem Rand des Mediums verbunden wird, und wobei das Oberteil mit dem verbundenen scheibenförmigen Medium von dem ersten Unterteil abgehoben und auf ein weiteres Unterteil einer weiteren mit dem Oberteil korrespondierenden Behandlungsvorrichtung aufgesetzt wird.

### Stand der Technik

In der Analytik von Flüssigkeiten und Gasen haben sich verschiedene Behandlungsmethoden etabliert, die poröse Medien, wie Filter und Membranen, einsetzen. Z.B. hat sich das Filtrationsverfahren zur Abreicherung und Konzentrierung von gelösten oder partikulären Inhaltsstoffen etabliert. Diese Konzentrierung ist meist notwendig, da die Konzentrationen der Verunreinigungen zu gering sind, um direkte Auswertungen durchzuführen. Die Filtrationsmethoden dienen als Vorstufe für weitere analytische Methoden, wie optische Auswertungen, sowie für weitere physikalische und chemische Reaktionen zur Signalverstärkung.

Aus der DE 198 23 993 ist eine erste Behandlungsvorrichtung, nämlich eine Einwegvorrichtung zur Keimzahlbestimmung bekannt, die einen Aufgusstrichter 1 aufweist, in dem ein Membranträger 10 klemmend gehalten wird, der von einer verklebten oder verschweißten Membran 21 verschlossen ist. Nach Entfernung einer Membranunterstützungseinheit kann der Membranträger 10 mit der Membran 21 über eine am Aufgusstrichter 1 angeordnete Auswerfvorrichtung 5 vom Aufgusstrichter 1 gelöst werden.

### Nachteilig dabei ist, dass eine spezielle

Behandlungsvorrichtung bzw. ein spezieller Auswurftrichter mit einer Auswurfvorrichtung notwendig ist. Für eine nachfolgende Aufnahme in einer weiteren Behandlungsvorrichtung, beispielsweise für eine weitere Filtration, ist die bekannte Vorrichtung nicht geeignet.

Auch aus der DE 10 2005 008 220 B3 ist eine Behandlungsvorrichtung bzw. Filtrationseinheit bekannt, die ein scheibenförmiges Medium bzw. einen Membranfilter aufweist, der zur weiteren Behandlung aus der Filtrationseinheit entnommen werden kann. Zur Entnahme des Mediums ist ein spezieller aufsteckbarer Entnahmering vorgesehen, auf dem der Membranfilter/das Medium festgeklemmt wird. Nach dem Entfernen des Entnahmerings kann dieser aufgespreizt werden, wodurch der Membranfilter freigegeben wird.

Nachteilig ist auch hier, dass eine spezielle Behandlungsvorrichtung bzw. Filtrationseinheit notwendig ist, die einen entsprechenden Ansatz für einen Entnahmering aufweist. Zudem ist es auch hier schwierig, das scheibenförmige Medium einer weiteren Behandlungsvorrichtung, beispielsweise einer weiteren Filtrationseinheit zuzuführen. Aus der DE 10 2008 005 968 A1 ist eine Nährmedieneinheit und ein Verfahren zur Aufnahme eines Filters aus einer Filtrationsvorrichtung bekannt. Die Nährmedieneinheit besteht dabei aus einem Deckel, der die eigentliche Transfereinheit bildet, und einem mit Nährmedium gefüllten Unterteil. Das als Deckel ausgebildete Oberteil weist dabei einen Fixierrand auf, der zur Entnahme des Filters bzw. Mediums aus der Filtrations- oder Behandlungsvorrichtung mit einem Rand des Filters über eine Klebehaftung verbindbar ist.

Aus der DE 10 2008 005 968 A1 ist weiterhin ein Verfahren zur mikrobiologischen Untersuchung flüssiger Proben bekannt, bei dem ein Oberteil bzw. Deckel einer Nährmedieneinheit auf einen in einem Unterteil einer Filter- bzw. Behandlungsvorrichtung angeordneten, als Membranfilter ausgebildeten Filter mit einem Fixierrand aufgesetzt wird. Dabei wird der Fixierrand des Deckels über eine Haftschicht mit einem Rand des Filters verbunden. Anschließend wird das Oberteil mit dem Filter von einer Filterunterstützung des Unterteils der Filtervorrichtung abgehoben und auf einer Oberfläche eines in dem Unterteil einer Nährmedieneinheit angeordneten Nährbodens abgelegt, wobei das Oberteil bzw. der Deckel das schalenförmig ausgebildete Unterteil abdeckt.

Nachteilig bei der bekannten Nährmedieneinheit und dem Verfahren, die sich für klassische, mikrobiologische Membrananwendungen, in denen man lediglich entstandene Kolonien visuell auswertet, bewährt haben, ist aber, dass sofort nach dem Transfer keine weitere Filtration oder Nachbehandlung direkt angeschlossen werden kann, ohne den Filter wieder von dem Deckel bzw. der Transfereinheit zu lösen. Bei dem bekannten Verfahren, bei dem sich die Filterfixierung in Kontakt mit Flüssigkeit zeitversetzt selbstständig wieder lösen kann, ist ein weiterer zeitversetzter Behandlungsschritt notwendig, um die gelöste Filtermembran wieder aufzugreifen und sie anderen Behandlungen zuzuführen oder sie in andere Einheiten einzufügen.

Diese Nachteile sind insbesondere bei Anwendungen von Bedeutung, die nicht dem klassischen Wachstum zurückgehaltener Keime auf Agar entsprechen, sondern die moderne molekularbiologische Markierungs- und Detektionsmethoden einsetzen, gleichgültig, ob es sich um Methoden handelt, die eine Vorinkubation benötigen oder direkt durch Markierungs- oder Verstärkungsmethoden untersucht werden.

### Aufgabenstellung

Aufgabe der vorliegenden Erfindung ist es daher, eine Vorrichtung und ein Verfahren bereitzustellen, bei dem es möglich ist, insbesondere ein poröses, scheibenförmiges Medium ohne komplizierte Vorrichtung und ohne den Gebrauch eines zusätzlichen Hilfsmittels einfach und kostengünstig in eine weitere Behandlungsstation einzubringen.

Die die Vorrichtung betreffende Aufgabe wird in Verbindung mit dem Oberbegriff des Anspruches 1 dadurch gelöst, dass das Oberteil eine mit einem abnehmbaren Deckel verschließbare Öffnung aufweist, über die eine Folgebehandlung in einer weiteren Behandlungsvorrichtung durchführbar ist, und dass der Fixierrand mit dem Rand des Mediums über eine Klebehaftung verbindbar ist.

Durch den abnehmbaren Deckel ist somit auch die Oberfläche des scheibenförmigen Mediums für weitere Bearbeitungsschritte zugänglich. Die beidseitige Zugänglichkeit ist insbesondere für poröse Medien von Bedeutung, da diese eine Behandlungsmöglichkeit sowohl von ober- als auch von unterhalb des porösen Mediums aufweisen. Aber auch dichte Membranen und Folien können mit dieser Transfereinheit transferiert werden.

Gemäß einer bevorzugten Ausführungsform der Erfindung bildet das Oberteil eine umlaufende Kontur, die mit den Behandlungsvorrichtungen korrespondiert, die in die Öffnung des Oberteils einsetzbar sind und/oder die mit Behandlungsvorrichtungen korrespondiert, auf die das Oberteil aufsetzbar ist. Dadurch wird einfach und kostengünstig eine Nachbehandlung des Mediums in unterschiedlichen Behandlungsvorrichtungen ermöglicht.

Nach einer weiteren bevorzugten Ausführungsform der Erfindung weist der Fixierrand des Oberteils eine Haftschicht aus einem geeigneten Kleber auf. Grundsätzlich ist es aber auch möglich, dass die Haftschicht aus einem geeigneten Kleber an dem Rand des scheibenförmigen Mediums angeordnet ist.

Durch die Haftschicht haftet das scheibenförmige Medium an dem Oberteil und lässt sich leicht und einfach aus der Behandlungsvorrichtung entnehmen und in eine weitere Behandlungsvorrichtung einbringen. Das scheibenförmige Medium, das auf die vorgesehene Behandlungsvorrichtung abgestimmt ist, ist meist rotationssymmetrisch ausgebildet, kann aber auch eine Rechteck- oder eine andere Vieleckstruktur aufweisen, sowie geometrisch unregelmäßige Formate. Die korrespondierende Transfereinheit bzw. das Oberteil ist in seiner umlaufenden Kontur funktional auf die Kontur bzw. Struktur des scheibenförmigen Mediums so abgestimmt, dass sie nur in dem Bereich einen Klebkontakt herstellt, der nicht zur weiteren Analyse verwendet wird.

Die Haftschicht ist aus einem PSA-Dispersionsklebstoff oder aus Acrylat-Copolymer-Mikrokugeln ausgebildet. Dadurch sind auch nasse Filter ausreichend am Fixierrand des Deckels anhaftbar und auch wieder abziehbar. Geeignete druckempfindliche Kleber sind dem Fachmann beispielsweise als mikrokugelbasierte Acrylatkleber bekannt.

Nach einer weiteren Ausführungsform der Erfindung ist der Kleber durch gängige Methoden sterilisierbar. Der Kleber ist bevorzugt DNA- und proteinfrei und zeigt keine unspezifischen Reaktionen mit antikörperbasierten Reagenzien. Insbesondere können Kleber verwendet werden, die nur geringe Autofluoreszenzen aufweisen und nicht unspezifisch mit entsprechenden Färbe- und Markierungsreagenzien binden. Dies gilt insbesondere für in der Auswertung gängige Wellenlängenbereiche von 400 bis 800 nm. Auch können Kleber verwendet werden, die keine antibiotischen oder fungiziden Eigenschaften aufweisen.

Gemäß einer weiteren bevorzugten Ausführungsform der Erfindung ist das scheibenförmige Medium als ein Filter und die erste Behandlungsvorrichtung als eine Filtervorrichtung ausgebildet. Der beispielsweise als poröser Membranfilter ausgebildete Filter kann dabei problemlos der Filtervorrichtung entnommen und einer weiteren Behandlungsvorrichtung zugeführt werden.

Nach einer weiteren bevorzugten Ausführungsform der Erfindung wird der Fixierrand von einer freien Stirnfläche einer an einer einem Unterteil der Behandlungsvorrichtung zugewandten Oberteilinnenfläche angeordneten Innenwandung gebildet. Die beispielsweise ringförmige Innenwandung stellt dabei zum einen den notwendigen Abstand zwischen scheibenförmigem Medium und Oberteil sicher und zum anderen ist ihr Durchmesser in der Dimension des Mediumdurchmessers so gestaltet, dass das Medium nur an seinem außenliegenden Rand mit der Haftschicht der Klebehaftung in Berührung kommt, wobei der Rand außerhalb der benutzten Fläche des Mediums angeordnet ist.

Gemäß einer weiteren bevorzugten Ausführungsform wird der Fixierrand von der Außenwand einer an einer einem Unterteil der Behandlungsvorrichtung zugewandten Oberteilinnenfläche angeordneten Innenwandung gebildet, wobei die Innenwandung beispielsweise als ringförmiges Klemmteil ausgestaltet ist, welches mit dem Medium über einen Verstärkungsrand des Mediums verklemmbar ist. Eine derartige Fixierung durch ein ringförmiges Klemmteil offenbart die DE 10 2007 014 082 A1.

Nach einer weiteren bevorzugten Ausführungsform der Erfindung weist das Oberteil an seiner Außenwandung eine Positionierungshilfe gegenüber der Behandlungsvorrichtung bzw. dem Unterteil der Behandlungsvorrichtung oder einer Analysevorrichtung auf. Dadurch werden wiederholbar eine reproduzierbare, ortsspezifische Behandlung und Auswertung ermöglicht, die auch automatisch durchgeführt werden können.

Die Positionierungshilfe ist vorzugsweise als mechanische Ausformung ausgebildet, die eine reproduzierbare Positionierung zwischen Oberteil und Behandlungsvorrichtung mit korrespondierender Ausformung und damit auch zwischen dem an dem Oberteil anhaftenden Medium und der Behandlungsvorrichtung erlaubt. Grundsätzlich sind auch andere Markierungen, z.B. Druck oder Ätzen im Bereich der Außenwandung des Oberteiles, möglich.

Gemäß einer weiteren bevorzugten Ausführungsform der Erfindung ist der in die Öffnung des Oberteils einsetzbare Deckel transparent ausgebildet. Dies ermöglicht bei bestimmten optischen Auswerteverfahren eine Auswertung bei geschlossenem Deckel.

Nach einer weiteren bevorzugten Ausführungsform der Erfindung weist das Oberteil einen durch seine Außenwandung begrenzten Aufnahmeansatz auf, der dichtend auf ein korrespondierendes Unterteil aufsetzbar ist und wobei die deckelseitige Öffnung durch einen einsetzbaren Deckel ebenfalls abdichtbar ist.

In vielen Fällen werden die Nachbehandlungsschritte auf wässriger Basis durchgeführt, wobei eine dichte Verbindung die Abführung überschüssigen Volumens, sei es durch Vakuum oder Druck, erlaubt.

Gemäß einer weiteren bevorzugten Ausführungsform der Erfindung ist ein schalenförmig ausgebildetes Unterteil als Teil der Transfereinheit vorgesehen, welches einen Nährboden aufweist, auf dessen dem Oberteil zugewandter Oberseite das am Deckel haftende scheibenförmige Medium ablegbar ist.

Die aus dem Oberteil mit Deckel und aus dem Unterteil bestehende Transfereinheit kann dabei als Nährmedieneinheit zur Aufnahme des scheibenförmigen Mediums, zu seiner Aufbewahrung und zu seiner Nachbehandlung verwendet werden. Die weitere Aufgabe bezüglich des Verfahrens wird in Verbindung mit dem Oberbegriff des Anspruches 13 dadurch gelöst, dass der Fixierrand mit dem Rand des Mediums über eine Klebehaftung verbunden wird und dass durch eine Öffnung im Oberteil mindestens eine weitere Untersuchung und/oder Behandlung der dem scheibenförmigen Medium anhaftenden Probenbestandteile erfolgt.

Durch die Nachbehandlung durch eine Öffnung im Oberteil kann auf ein zeitraubendes Ablösen des scheibenförmigen Mediums von dem Oberteil verzichtet werden. Dadurch werden Nachfolgebehandlungen mit unterschiedlichen Behandlungsvorrichtungen erheblich vereinfacht und

Die weitere Aufgabe bezüglich des Verfahrens wird in Verbindung mit dem Oberbegriff des Anspruches 15 dadurch gelöst, dass durch eine Öffnung im Oberteil mindestens eine weitere Untersuchung und/oder Behandlung der dem scheibenförmigen Medium anhaftenden Probenbestandteile erfolgt.

Durch die Nachbehandlung durch eine Öffnung im Oberteil kann auf ein zeitraubendes Ablösen des scheibenförmigen Mediums von dem Oberteil verzichtet werden. Dadurch werden Nachfolgebehandlungen mit unterschiedlichen Behandlungsvorrichtungen erheblich vereinfacht und verbessert. Ein in die Öffnung im Oberteil eingesetzter, die Öffnung verschließender Deckel kann vor der weiteren Untersuchung und/oder Behandlung der dem scheibenförmigen Medium anhaftenden Probenbestandteile abgenommen werden.

Nach einer weiteren bevorzugten Ausführungsform der Erfindung wird vor dem ersten Einsatz des Oberteils eine das Oberteil an seiner der Deckelöffnung abgewandten Seite steril versiegelnde Abdeckung abgezogen.

Damit kann die Transfereinheit, bestehend aus dem Oberteil mit eingesetztem Deckel, nach unten durch eine versiegelnde Abdeckung abgeschlossen, in einer sterilen Verpackungshülle geliefert werden. Vor dem ersten Einsatz muss dann lediglich die Transfereinheit ausgepackt und die versiegelnde Abdeckung, die beispielsweise als eine anhaftende Folie ausgebildet ist, abgezogen werden.

Anstelle der die Transfereinheit versiegelnden Abdeckung kann die Transfereinheit auch mit einem schalenförmigen Unterteil geliefert werden.

Der Deckel des Oberteils dient dabei als Verschluss, um einerseits die Sterilität zu gewährleisten und um andererseits z.B. die Probe vor äußeren Einflüssen zu schützen.

Damit ergibt sich ein einfaches und kostengünstiges Manipulieren des Mediums mit der aufgebrachten Probe.

Nach einer weiteren Ausführungsform der Erfindung wird das als Filterscheibe ausgebildete Medium, z.B. eine Membran, nach einer Filtration in der ersten Behandlungsvorrichtung sowie optionalen weiteren Behandlungsschritten zur Signalverstärkung mit der Transfereinheit auf eine feste Unterstützungsscheibe, die ebenfalls eine Haftschicht aus einem geeigneten Kleber aufweist, aufgebracht und in einem Folgeschritt mit der Unterstützungsscheibe in eine automatische Auswerteeinheit für eine automatische Auswertung eingesetzt.

Über die flächige Fixierung mit der Unterstützungsscheibe wird sichergestellt, dass der Auswertevorgang über eine definierte Fokussierungsebene erfolgt. Die Auswertung kann hierbei entweder direkt auf der Membranfläche oder durch die hochtransparente Unterstützungsscheibe erfolgen.

Damit wird es möglich, das scheibenförmige Medium mit elektronischen Auswerteeinheiten, wie z.B. Laser-Scannern, auszuwerten.

So müssen z.B. für eine mikrobiologische Analyse Porengrößen im Bereich der Mikrofiltration verwendet werden, um die Mikroorganismen sicher zurückhalten zu können. Da die Filtrationsflüsse proportional mit der Dicke des Mediums bzw. Filters und kleiner werdendem Porendurchmesser abnehmen, werden die Filtrationsmedien dünn ausgeführt. Typisch sind für scheibenförmige poröse Medien Dicken von ca. 100 bis 200 µm. Solche Filtrationsmedien können nicht ohne Unterstützung in Auswerteeinheiten mit Laser-Scanner-Technik eingesetzt werden.

Unter Benutzung der erfindungsgemäßen Transfereinheit kann ein als Filterscheibe ausgebildetes Medium nach erfolgter Filtration und den notwendigen Behandlungsschritten auf eine feste Unterstützungsscheibe aufgebracht werden.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:
- Figur 1:: eine Seitenansicht einer Transfereinheit, bestehend aus Oberteil mit Deckel und einer steril versiegelnden Abdeckung im Schnitt;
- Figur 2:: eine Seitenansicht des Deckels von Figur 1 im Schnitt;
- Figur 3:: eine Seitenansicht des Oberteils von Figur 1 im Schnitt;
- Figur 4:: eine Untersicht auf die Transfereinheit von Figur 1 aus Richtung IV;
- Figur 5:: eine Seitenansicht einer zur Nachbehandlung in eine Behandlungsvorrichtung eingesetzten Transfereinheit im Schnitt;
- Figur 6:: eine als Filtrationsvorrichtung ausgebildete Behandlungsvorrichtung im Schnitt;
- Figur 7:: eine Seitenansicht im Schnitt eines auf das Unterteil mit scheibenförmigem Medium von Figur 6 aufgesetzten Oberteils einer Transfereinheit mit Deckel und
- Figur 8:: eine Seitenansicht im Schnitt des Oberteils von Figur 7 mit scheibenförmigem Medium aufgesetzt auf ein Unterteil einer Transfereinheit.

### Beschreibung der Ausführungsbeispiele

Eine Transfereinheit 1 besteht im Wesentlichen aus einem Oberteil 2 und einem Deckel 3.

Das Oberteil 2 bildet eine umlaufende Kontur mit einer Außenwandung 4 und einer parallel verlaufenden Innenwandung 5, die eine Öffnung 6 im Oberteil 2 umschließt. Auf seiner Oberseite 7 weist das Oberteil 2 einen Absatz 8 auf, der die Öffnung 6 umschließt und den Deckel 3 mit einem Ansatz 9 aufnimmt. Das Oberteil 2 weist an seiner der Oberseite 7 abgewandten Oberteilinnenfläche 10 die Innenwandung 5 auf, deren freies Ende mit seiner Stirnfläche einen Fixierrand 11 bildet. In den Ausführungsbeispielen weist der Fixierrand 11 eine Haftschicht 12 aus einem geeigneten Klebestoff auf. Die Haftschicht 12 ist beispielsweise aus einem PSA-Dispersionsklebstoff oder aus Acrylat-Copolymer-Mikrokugeln ausgebildet. Der abnehmbare Deckel 3 ist mit seinem Ansatz 9 in den Absatz 8 in das Oberteil 2 dichtend einsetzbar. Die dichtende Einsetzbarkeit kann z. B. durch konische Schrägen oder durch eine umlaufende Klemmung in Abhängigkeit vom Material von Deckel 3 bzw. Oberteil 2 gewährleistet werden. Der Deckel 3 ist aus einem transparenten Kunststoff ausgebildet.

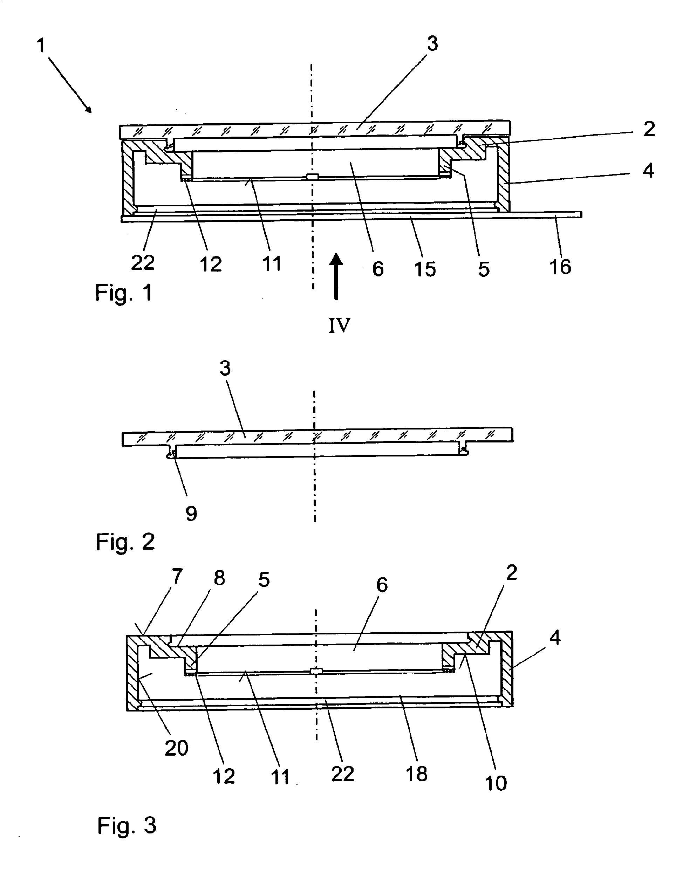

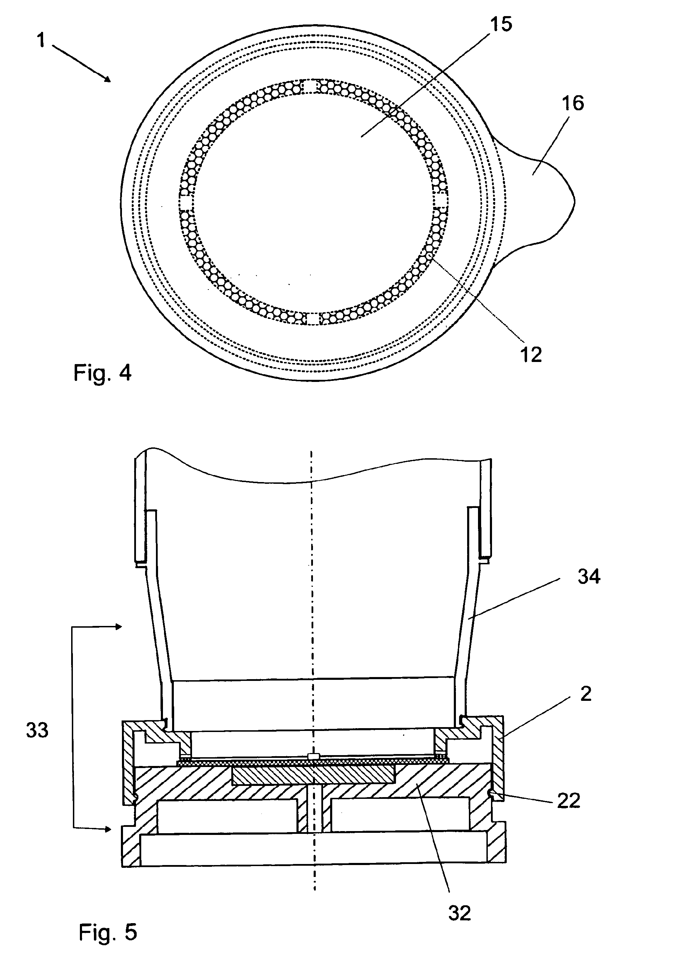

Entsprechend dem Ausführungsbeispiel der Figuren 1 und 4 weist das Oberteil 2 an seinem freien Ende der Außenwandung 4 eine versiegelnde Abdeckung 15 auf, die über eine Lasche 16 von dem Oberteil 2 bzw. der Außenwandung 4 abziehbar ist.

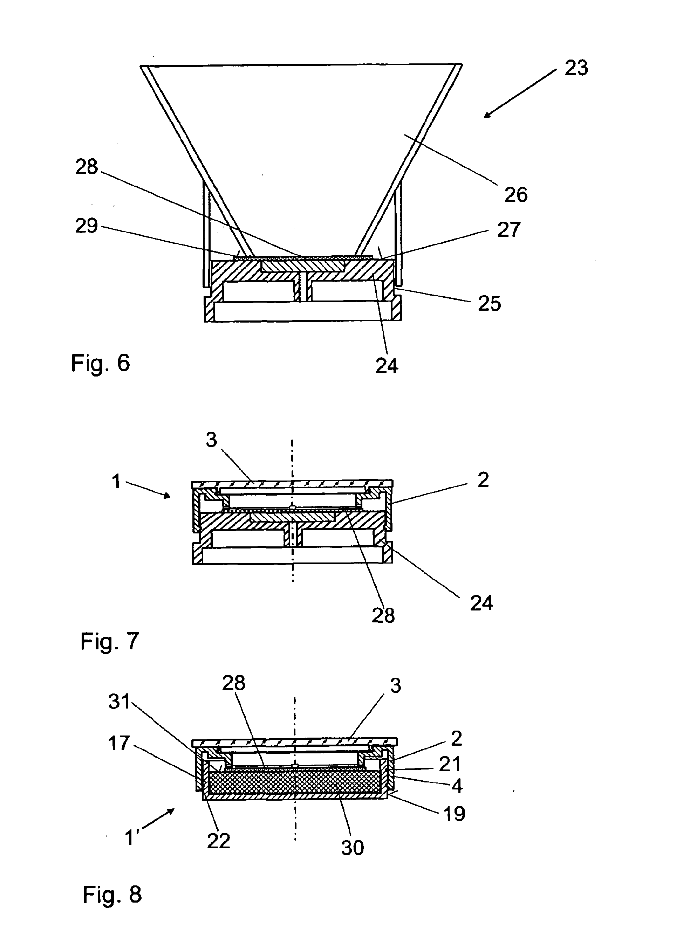

Anstelle der Abdeckung 15 kann die Transfereinheit 1' auch entsprechend dem Ausführungsbeispiel von Figur 8 auch ein Unterteil 17 aufweisen, auf das das Oberteil 2 mit einem durch seine Außenwandung 4 seitlich begrenzten Aufnahmeansatz 18 aufsetzbar ist. Dabei kann eine Außenwandung 19 des Unterteils 17 an der Innenseite 20 der Außenwandung 4 anliegen.

Eine an sich bekannte erste Behandlungsvorrichtung 23, die entsprechend Figur 6 als eine Filtrationsvorrichtung ausgebildet ist, besteht aus einem Unterteil 24 mit einem Aufnahmeabsatz 25, auf dem ein trichterförmiger Aufsatz 26 aufsetzbar ist. Zwischen dem Aufsatz 26 und einer Filterunterstützungsfläche 27 des Unterteils 24 ist ein scheibenförmiges Medium 28, das beispielsweise als eine poröse Filtermembran ausgebildet ist, angeordnet.

Nach einem Filtrationsvorgang kann der Aufsatz 26 von dem Unterteil 24 abgenommen werden und das Oberteil 2 der Transfereinheit 1, 1' kann mit eingesetztem Deckel anstelle des Aufsatzes 26 auf das Unterteil 24 aufgesetzt werden. Dabei wird das Oberteil 2 mit seinem Fixierrand 11 auf einen Rand 29 des scheibenförmigen Mediums 28 aufgesetzt, so dass das scheibenförmige Medium 28 an der Haftschicht 12 des Fixierrandes 11 haftet und von dem Unterteil 24 aufgenommen werden kann. Die Transfereinheit 1, 1' bzw. das Oberteil 2 kann nunmehr auf das schalenförmig ausgebildete Unterteil 17, in das ein Nährboden 30 eingebracht wurde, aufgesetzt werden, wobei das scheibenförmige Medium 28 auf einer Oberseite 31 des Nährbodens 30 aufliegt. Nach Abnahme des Deckels 3 von dem Oberteil 2 können durch die Öffnung 6 weitere Behandlungsschritte vorgenommen werden. Die Transfereinheit 1, 1' bzw. das Oberteil 2 kann mit dem scheibenförmigen Medium 28 auch auf ein Unterteil 32 einer weiteren Behandlungsvorrichtung 33 aufgesetzt werden.

Gemäß Figur 5 kann nach Abnahme des Deckels 3 in die Öffnung 6 ein Trichteraufsatz 34 der weiteren Behandlungsvorrichtung 33 oder eine andere nicht dargestellte Behandlungsvorrichtung dichtend eingesetzt werden. Die dichtende Aufsetzbarkeit des Oberteils 2 auf Unterteil 32 sowie des Trichteraufsatzes 34 auf Oberteil 2 kann z. B. durch konische Schrägen oder durch eine umlaufende Klemmung in Abhängigkeit vom Material von Unterteil 32, Oberteil 2 und Trichteraufsatz 34 gewährleistet werden. Gemäß Figur 5 kann eine umlaufende Dichtwulst 22 in eine korrespondierende, ebenfalls umlaufende Vertiefung des Unterteils 32 eingreifen.

## Patentansprüche

1. Transfereinheit (1, 1') zur Aufnahme eines insbesondere porösen, scheibenförmigen Mediums (28) aus einer ersten Behandlungsvorrichtung (23), mit mindestens einem Oberteil (2), wobei das Oberteil (2) einen Fixierrand (11) aufweist, der zur Entnahme des Mediums (28) aus der ersten Behandlungsvorrichtung (23) mit einem Rand des Mediums (28) verbindbar ist,
**dadurch gekennzeichnet,**
**dass** das Oberteil (2) eine mit einem abnehmbaren Deckel (3) verschließbare Öffnung (6) aufweist, über die eine Folgebehandlung in einer weiteren Behandlungsvorrichtung (33) durchführbar ist, und
**dass** der Fixierrand (11) mit dem Rand des Mediums (28) über eine Klebehaftung verbindbar ist.

2. Transfereinheit nach Anspruch 1,
**dadurch gekennzeichnet,**
**dass** das Oberteil (2) eine umlaufende Kontur bildet, die mit den Behandlungsvorrichtungen (23, 33) korrespondiert, die in die Öffnung (6) des Oberteils (2) einsetzbar sind und/oder die mit Behandlungsvorrichtungen (23, 33) korrespondiert, auf die das Oberteil (2) aufsetzbar ist.

3. Transfereinheit nach Anspruch 1 oder 2,
**dadurch gekennzeichnet,**
**dass** der Fixierrand (11) des Oberteils (2) eine Haftschicht (12) aus einem geeigneten Kleber aufweist.

4. Transfereinheit nach Anspruch 1 oder 2,
**dadurch gekennzeichnet,**
**dass** der Rand (29) des scheibenförmigen Mediums (28) eine Haftschicht (12) aus einem geeigneten Kleber aufweist.

5. Transfereinheit nach Anspruch 3 oder 4,
**dadurch gekennzeichnet,**
**dass** der Kleber sterilisierbar ausgebildet ist.

6. Transfereinheit nach einem der Ansprüche 3 bis 5,
**dadurch gekennzeichnet,**
**dass** der Kleber DNA- und proteinfrei ausgebildet ist und keine unspezifischen Reaktionen mit antikörperbasierten Reagenzien zeigt.

7. Transfereinheit nach einem der Ansprüche 1 bis 6,
**dadurch gekennzeichnet,**
**dass** das scheibenförmige Medium (28) als ein Filter und die erste Behandlungsvorrichtung (23) als eine Filtervorrichtung ausgebildet ist.

8. Transfereinheit nach einem der Ansprüche 1 bis 7,
**dadurch gekennzeichnet,**
**dass** der Fixierrand (11) von einer freien Stirnfläche einer an einer einem Unterteil (17) der Behandlungsvorrichtung (23) zugewandten Oberteilinnenfläche (10) angeordneten Innenwandung (5) gebildet ist.

9. Transfereinheit nach einem der Ansprüche 1 bis 8,
**dadurch gekennzeichnet,**
**dass** das Oberteil (2) an seiner Außenwandung (4) eine Positionierungshilfe gegenüber der Behandlungsvorrichtung (23, 33) aufweist.

10. Transfereinheit nach einem der Ansprüche 1 bis 9,
**dadurch gekennzeichnet,**
**dass** der in die Öffnung (6) des Oberteils (2) einsetzbare Deckel (3) transparent ausgebildet ist.

11. Transfereinheit nach einem der Ansprüche 1 bis 10,
**dadurch gekennzeichnet,**
**dass** das Oberteil (2) einen durch seine Außenwandung (4) begrenzten Aufnahmeansatz (18) aufweist, der auf ein korrespondierendes Unterteil (17) aufsetzbar ist und dass ein in die Öffnung (6) eingesetzter Deckel (3) die Öffnung (6) abdichtet.

12. Transfereinheit nach einem der Ansprüche 1 bis 11,
**dadurch gekennzeichnet,**
**dass** ein schalenförmig ausgebildetes Unterteil (17) als Teil der Transfereinheit (1') vorgesehen ist, das einen Nährboden (30) aufweist, auf dessen dem Oberteil (2) zugewandter Oberseite (31) das am Deckel (3) haftende scheibenförmige Medium (28) ablegbar ist.

13. Verfahren zur mikrobiologischen Untersuchung und/oder Behandlung flüssiger Proben, bei dem ein Oberteil (2) einer Transfereinheit (1, 1') auf ein in einem ersten Unterteil (17) einer ersten Behandlungsvorrichtung (23) angeordnetes und der flüssigen Probe ausgesetztes scheibenförmiges Medium (28) so aufgesetzt wird, dass ein in dem Oberteil (2) angeordneter Fixierrand (11) mit einem Rand (29) des Mediums (28) (12) verbunden wird, und wobei das Oberteil (2) mit dem verbundenen scheibenförmigen Medium (28) von dem ersten Unterteil (17) abgehoben und auf ein weiteres Unterteil (32) einer weiteren mit dem Oberteil (2) korrespondierenden Behandlungsvorrichtung (33) aufgesetzt wird,
**dadurch gekennzeichnet,**
**dass** der Fixierrand (11) mit dem Rand 29) des Mediums (28) über eine Klebehaftung verbunden wird und
**dass** durch eine Öffnung (6) im Oberteil (2) mindestens eine weitere Untersuchung und/oder Behandlung der dem scheibenförmigen Medium (28) anhaftenden Probenbestandteile erfolgt.

14. Verfahren nach einem der Anspruch 13,
**dadurch gekennzeichnet,**
**dass** vor der weiteren Untersuchung und/oder Behandlung ein die Öffnung (6) verschließender Deckel (3) abgenommen wird.

15. Verfahren nach Anspruch 13 oder 14,
**dadurch gekennzeichnet,**
**dass** vor dem ersten Einsatz des Oberteils (2) eine das Oberteil (2) an seiner der Deckelöffnung (6) abgewandten Seite steril versiegelnde Abdeckung (15) abgezogen wird.

## Claims

1. A transfer unit (1, 1') for receiving an in particular porous disk-shaped medium (28) from a first treatment device (23), having at least one upper part (2), wherein the upper part (2) has a fixing edge (11), which can be connected to an edge of the medium (28) for removing the medium (28) from the first treatment device (23), **characterized in that** the upper part (2) has an opening (6) that can be closed with a removable lid (3), via which a subsequent treatment in a further treatment device (33) can be carried out, and that the fixing edge (11) can be connected to the edge of the medium (28) via an adhesive bond.

2. The transfer unit according to Claim 1, **characterized in that** the upper part (2) has a peripheral contour that corresponds to the treatment devices (23, 33), which can be inserted into the opening (6) of the upper part (2) and/or that corresponds to the treatment devices (23, 33) onto which the upper part (2) can be placed.

3. The transfer unit according to Claim 1 or 2, **characterized in that** the fixing edge (11) of the upper part (2) has an adhesive layer (12) of a suitable adhesive.

4. The transfer unit according to Claim 1 or 2, **characterized in that** the edge (29) of the disk-shaped medium (28) has an adhesive layer (12) of a suitable adhesive.

5. The transfer unit according to Claim 3 or 4, **characterized in that** the adhesive is embodied in a sterilizable manner.

6. The transfer unit according to any of Claims 3 through 5, **characterized in that** the adhesive is embodied in a DNA-free and protein-free manner, and does not exhibit any non-specific reactions with anti-body based reagents.

7. The transfer unit according to any of Claims 1 through 6, **characterized in that** the disk-shaped medium (28) is embodied as a filter and the first treatment device (23) is embodied as a filter device.

8. The transfer unit according to any of Claims 1 through 7, **characterized in that** the fixing edge (11) is formed by a free end face of an inner wall (5) arranged on an upper part inner surface (10) facing towards a lower part (17) of the treatment device (23).

9. The transfer unit according to any of Claims 1 through 8, **characterized in that** the upper part (2) on its outer wall (4) has a positioning aid with respect to the treatment device (23, 33).

10. The transfer unit according to any of Claims 1 through 9, **characterized in that** the lid (3) that can be inserted into the opening (6) of the upper part (2) is embodied in a transparent manner.

11. The transfer unit according to any of Claims 1 through 10, **characterized in that** the upper part (2) has a receiving projection (18) delimited by its outer wall (4), which receiving projection can be placed on a corresponding lower part (17), and **in that** a lid (3) inserted into the opening (6) seals the opening (6).

12. The transfer unit according to any of Claims 1 through 11, **characterized in that** a lower part (17) embodied in a dish-shaped manner is provided as a part of the transfer unit (1') and has a nutrient medium (30) on the upper side (31) of which facing towards the upper part (2) the disk-shaped medium (28) adhering to the lid (3) can be placed.

13. A method for the microbiological testing and/or treatment of liquid samples, in which an upper part (2) of a transfer unit (1, 1') is placed on a disk-shaped medium (28) arranged in a first lower part (17) of a first treatment device (23) and exposed to the liquid sample such that a fixing edge (11) arranged in the upper part (2) is connected to an edge (29) of the medium (28) (12) and wherein the upper part (2) is lifted with the connected disk-shaped medium (28) from the first lower part (17) and is placed on a further lower part (32) of a further treatment device (33) that corresponds to the upper part (2), **characterized in that** the fixing edge (11) is connected to the edge (29) of the medium (28) via an adhesive bond and that at least one further test and/or treatment of the sample constituents adhering to the disk-shaped medium (28) is carried out through an opening (6) in the upper part (2).

14. The method according to Claim 13, **characterized in that** a lid (3) closing the opening (6) is removed before the further test and/or treatment.

15. The method according to Claim 13 or 14, **characterized in that** a cover (15) sealing the upper part (2) in a sterile manner on its side facing away from the lid opening (15) is removed before the first use of the upper part (2).

## Revendications

1. Unité de transfert (1, 1') destinée à recevoir notamment un milieu en forme de disque (28) poreux provenant d'un premier dispositif de traitement (23), ladite unité de transfert ayant au moins une partie supérieure (2) disposant d'un bord de fixation (11), lequel peut être attaché à un bord du milieu (28) afin d'extraire le milieu (28) du premier dispositif de traitement (23), **caractérisée en ce que** la partie supérieure (2) présente une ouverture (6) - pouvant être fermée à l'aide d'un couvercle amovible (3) - à travers laquelle il est possible de réaliser un traitement ultérieur dans un autre dispositif de traitement (33), et **en ce que** le bord de fixation (11) peut être attaché au bord du milieu (28) par collage.

2. Unité de transfert selon la Revendication 1, **caractérisée en ce que** la partie supérieure (2) possède un contour périphérique adapté aux dispositifs de traitement (23, 33), lesquels peuvent être insérés dans l'ouverture (6) de la partie supérieure (2) et/ou sur lesquels la partie supérieure (2) peut être positionnée.

3. Unité de transfert selon la Revendication 1 ou 2, **caractérisée en ce que** le bord de fixation (11) de la partie supérieure (2) dispose d'une couche adhésive (12) composée d'une substance collante appropriée.

4. Unité de transfert selon la Revendication 1 ou 2, **caractérisée en ce que** le bord (29) du milieu en forme de disque (28) dispose d'une couche adhésive (12) composée d'une substance collante appropriée.

5. Unité de transfert selon la Revendication 3 ou 4, **caractérisée en ce que** l'adhésif est de nature à permettre la stérilisation.

6. Unité de transfert selon l'une quelconque des Revendications 3 à 5, **caractérisée en ce que** l'adhésif est exempt d'ADN et de protéine et ne présente pas de réaction non spécifique à des réactifs à base d'anticorps.

7. Unité de transfert selon l'une quelconque des Revendications 1 à 6, **caractérisée en ce que** le milieu en forme de disque (28) est intégré en tant que filtre, et le premier dispositif de traitement (23) est conçu sous forme de dispositif de filtrage.

8. Unité de transfert selon l'une quelconque des Revendications 1 à 7, **caractérisée en ce que** le bord de fixation (11) est formé par une face frontale libre d'une paroi interne (5) disposée sur une surface interne de la partie supérieure interne (10) positionnée face à une partie inférieure (17) du dispositif de traitement (23).

9. Unité de transfert selon l'une quelconque des Revendications 1 à 8, **caractérisée en ce que** la partie supérieure (2) dispose, sur sa paroi externe (4), d'un support de positionnement vis-à-vis du dispositif de traitement (23, 33).

10. Unité de transfert selon l'une quelconque des Revendications 1 à 9, **caractérisée en ce que** le couvercle (3) pouvant être inséré dans l'ouverture (6) de la partie supérieure (2) est conçu de façon transparente.

11. Unité de transfert selon l'une quelconque des Revendications 1 à 10, **caractérisée en ce que** la partie supérieure (2) comporte une saillie de réception (18) délimitée par sa paroi externe (4), ladite saillie de réception pouvant être placée sur une partie inférieure (17) correspondante, et **en ce que** l'insertion d'un couvercle (3) dans l'ouverture (6) scelle cette ouverture (6).

12. Unité de transfert selon l'une quelconque des Revendications 1 à 11, **caractérisée en ce qu'**une partie inférieure (17) en forme d'assiette est intégrée à l'unité de transfert (1') et dispose d'un milieu nutritif (30), sur la partie supérieure (31) duquel il est possible de placer le milieu en forme de disque (28) collé au couvercle (3), en le positionnant face à la partie supérieure (2).

13. Une méthode pour l'essai et/ou le traitement microbiologique d'échantillons liquides, selon laquelle une partie supérieure (2) d'une unité de transfert (1, 1') est placée sur un milieu en forme de disque (28), lequel est positionné dans une partie inférieure (17) d'un premier dispositif de traitement (23) et exposé à l'échantillon liquide de sorte qu'un bord de fixation (11) intégré à la partie supérieure (2) soit attaché à un bord (29) du milieu (28) (12) et que la partie supérieure (2) soit soulevée de la première partie inférieure (17) en même temps que le milieu en forme de disque (28) attaché et soit placée sur une autre partie inférieure (32) d'un autre dispositif de traitement (33) adapté à la partie supérieure (2), la méthode étant **caractérisée en ce que** le bord de fixation (11) est attaché au bord (29) du milieu (28) par collage et **en ce qu'**au moins un autre essai et/ou traitement des composants de l'échantillon adhérant au milieu en forme de disque (28) est réalisé à travers une ouverture (6) située dans la partie supérieure (2).

14. La méthode selon la Revendication 13, **caractérisée en ce qu'**un couvercle (3) fermant l'ouverture (6) est retiré avant de procéder à l'autre essai et/ou traitement.

15. La méthode selon la Revendication 13 ou 14, **caractérisée en ce qu'**un couvercle (15) scellant la partie supérieure (2) de façon stérile du côté opposé à l'ouverture du couvercle (15) est retiré avant la première utilisation de la partie supérieure (2).
